Europäisches Patentamt

European Patent Office

Office européen des brevets

(11) Publication number: 0 344 992
A1

## EUROPEAN PATENT APPLICATION

(21) Application number: 89305380.1

(51) Int. Cl.⁴: A01G 7/00

(22) Date of filing: 26.05.89

(30) Priority: 31.05.88 JP 131450/88
31.05.88 JP 131451/88

(43) Date of publication of application:
06.12.89 Bulletin 89/49

(84) Designated Contracting States:
AT BE CH DE ES FR GB GR IT LI LU NL SE

(71) Applicant: MITSUI PETROCHEMICAL
INDUSTRIES, LTD.
2-5, Kasumigaseki 3-chome Chiyoda-ku
Tokyo 100(JP)

(72) Inventor: Tanimoto, Shizufumi
1034, Honjo Aza Ipponsugi Honjo-cho
Saga-shi Saga-ken(JP)
Inventor: Hirata, Yukimasa Mitsui
Petrochemical Ind. Ltd.
1-2, Waki 6-Chome Waki-cho
Kuga-gun Yamaguchi-ken(JP)
Inventor: Takahashi, Shigeru Mitsui
Petrochemical Ind. Ltd.
1-2, Waki 6-Chome Waki-cho
Kuga-gun Yamaguchi-ken(JP)
Inventor: Ono, Reiko Mitsui Petrochemical
Ind. Ltd.
1-2, Waki 6-Chome Waki-cho
Kuga-gun Yamaguchi-ken(JP)

(74) Representative: Myerscough, Philip Boyd et al
J.A.Kemp & Co. 14, South Square Gray's Inn
London, WC1R 5EU(GB)

(54) Method for propagating nursery plant.

(57) A method for propagating a nursery plant by starting the cultivation of a tissue piece or a cultured cell of a plant on a medium prepared by impregnating a water-insoluble and water-absorbent support with a liquid culture medium in a culture tank and carrying out the subsequent cultivation consistently in the same culture tank and on the same medium, where the tissue piece or the cultured cell of a plant is heterotrophically cultivated on a carbon source-containing medium before the regeneration of a shoot and the shoot in the processes of rooting and acclimatization is photo-autotrophically cultivated on a substantially carbon source-free medium brought by the consumption of the carbon source during the heterotrophical cultivation period before the regeneration of the shoot, by making the shoot photosynthesize under a required light exposure intensity and required carbon dioxide gassing condition; and a method for acclimatizing a cultured nursery plant by cultivating it while making it photosynthsize in a carbon source-free medium under a required light exposrue intensity and required carbon dioxide gassing conditions in a culture tank and, at the same time, gradually decreasing the moisture of the acclimatization medium and the humidity in the culture tank by gassing carbon dioxide into the culture tank during the acclimatizing cultivation period to thereby discharge the indide moisture outside.

Xerox Copy Centre

# FIG.1

(A)    (B)    (C)    (D)    (E)    (F)

## METHOD FOR PROPAGATING NURSERY PLANT

BACKGROUND OF THE INVENTION

This invention relates to a method for propagating a nursery plant and a method for acclimatizing a cultured nursery plant. In further detail, this invention relates to a method for propagating a nursery plant by carrying out the cultivation in all the processes of regeneration, rooting and acclimatization of a shoot consistently in the same culture tank and on the same culture medium and a method for acclimatizing a cultured nursery plant which is preferably applicable in the consistent cultivation.

A method for propagating a nursery plant by tissue culture is a technique effective for the production of a high-yielding and high quality nursery plant by the use of a nursery plant which is not contaminated with pathogens such as virus and the like, the mass propagation of cloned nursery plants (genetically homogenous nursery plant) of a new cultivar, a slightly germinative seed crop, a slightly vegetatively propagative crip, etc., and it is considered that this method will grow into one of important supports for the future agricultural production techniques. However, the cost for producing nursery plants by cultivation of cells, tissues or plantlets of a plant in a culture tank is high, so that the spread of this method is restricted to a part of high class protected horticulture crops such as an orchid, a strawberry, a carnation, an ornamental foliage plant, etc. The greatest reason why the production cost becomes high is that these high class protected horticulture crops are produced mostly by manual industry and thus the labor cost accounts for 65 to 70% of the production cost. The secondary reason is that the growth of cells, tissues or plantlets of a plant during the tissue culture period is late and the yield of nursery plants having marketability among those obtained thereby and preferable root taking ability and growing ability in the subsequent acclimatization process is low.

On the other hand, it has been so far considered practically impossible to consistently make a plantlet during tissue culture grow autotrophically to thereby produce a nursery plant because the plantlet during tissue culture considerably loses photsynthesizing ability. It can be said the establishment of such a fixed idea as above is owing to that plant tissue culture techniques have been developed based upon the cell or tissue culture techniques of a micro-organism or an animal. Thus, it was a popular common knowledge in the prior arts that a plantlet was grown by adding a sugar (sucrose, glucose, fructose, etc.) as a carbon source to a culture medium to make the plantlet do mixed vegetative growth showing a marked aspect of heterotrophical growth. As the result, the research developments of the improvement in productivity and the reduction of production cost in the prior arts were both promoted with the aims of increasing the efficiency of heterotrophical growth and propagation to the utmost limit and reducing labors therefor. As a typical example of research developments having the above lines, a method of increasing the speed of absorption of sugar, oxygen, etc. into a plantlet by immersing most of the plantlet into a sugar-containing liquid medium in a culture tank and shaking or rotating the culture tank while supplying oxygen thereto and like methods can be enumerated.

In the conventional methods as above, a medium is apt to be contaminated from the outside during the cultivation period because of the addition of a large quantity of sugars, so that there often arises a trouble of a plantlet under cultivation being putrified to death by the contamination during the cultivation period. In order to prevent this trouble, the conventional methods require such a strict handling as to keep cultivation environments such as a culture tank, a cultivation chamber, etc. substantially sterile. In addition, it is considered that the transfer of a cultured nursery plant produced mainly by such heterotrophical growth and propagation as above to acclimatization process which is under autotrophical conditions gives an abrupt environmental change to the cultured nursery plant to bring a cause of the lowering of root taking ratio and the delay of growth in the acclimatization process.

Furthermore, the conventional methods adopt a way in which a cultured nursery plant is acclimatized gradually so as to become adaptable to the outside environment, particularly by carrying out the acclimatization of a nursery plant in an apparatus which is different from that of the precedent cultivation process and then stepwise lowering the inside humidity of the apparatus and like means.

However, such an acclimatization process as above could be a major obstacle in putting the mass propagation of a nursery plant by tissue culture into operation industrially and efficiently.

In addition, a cultured nursery plant is still in an environment showing a fairly marked heterotrophical aspect in the conventional acclimatization process, so that it is feared that even a cultured nursery plant undergone the acclimatization process cannot adapt sufficiently to a complete photo-autotrophical environment after setting.

## SUMMARY OF THE INVENTION

The object of the present invention lies in providing a novel method for propagating a nursery plant and a novel method for acclimatizing a cultured nursery plant, both of which are free from the problems of the above prior arts. More specifically, the present invention purposes to provide a method for propagating a nursery plant, by which the propagation of a nursery plant can be carrid out through the cultivation in all the processes of regeneration, rooting and acclimatization of a shoot consistently in the same culture tank and on the same medium, the root taking ration at the tim of acclimatization and the growth of a nursery plant after acclimatization can be preferable and the restrictions on handling for keeping a sterile state where the contamination during the cultivation period hardly takes place are relaxed, and a method for the acclimatization, which requires no special apparatus for acclimization and thus is suitable for a process of acclimatization in the foregoing consistent cultivaticn.

The foregoing first object is attained by carrying out the cultivation of a tissue piece or a cultured cell of a plant in a state of its being placed on a culture medium prepared by impregnating a water-insoluble and water-absorbent support with a liquid culture medium in a culture tank and the subsequent cultivation of a regenerated shoot consistently in the same culture tank and on the same culture medium, and, in this consistent cultivation, carrying out the cultivation before the shoot regeneration heterotrophically in a carbon source-containing culture medium and that after the shoot regeneration photo-autotrophically by making the regenerated shoot photosynthesize under a required light exposure intensity and required carbon dioxide gassing conditions in the culture medium in which the foregoing carbon source has been substantially consumed in the foregoing heterotrophical cultivation period.

The foregoing second object is attained by supplying carbon dioxide into the culture tank under required conditions during the photo-autotrophical cultivation period of a cultured nursery plant.

According to the present method for propagating a nursery plant, the following striking effects can be obtained. That is, a nursery plant can be propagated by carrying out the cultivation consistently in the same culture tank and on the same culture medium to considerably simplify the whole step. In addition, a shoot in processes of rooting and acclimatization after the regeneration of the shoot is cultivated photo-autotrohpically to make the rooting ratio, the root taking ratio after acclimatization, the growth of a nursery plant after acclimatization, etc. preferable, so that the yield of superior nursery plants is sharply improved. Furthermore, the contamination during the cultivation period is controlled to relax the management for keeping a culture tank sterile.

Furthermore, according the present method for acclimatizing a cultured nursery plant, the decrease of the humid ity in the culture tank as is considered essential in the acclimatization process is simultaneously carried out by the gassing of carbon dioxide into a culture tank as a requisite for giving photo-autotrophical conditions, so that it becomes unnecessary to use a special apparatus for acclimatization. In addition, this method can be employed as a part of cultivation process of a cultured nursery plant in the same culture tank and on the same medium. Furthermore, the present acclimatization method strikingly improves the rooting ratio of a cultured nursery plant.

## BRIEF DESCRIPTION OF THE DRAWINGS

Fig. 1 is a schematic flow chart showing Steps (1) to (6) during the cultivation period according to the present method for acclimatizing a cultured nursery plant.

Fig. 2 illustrates perspective views showing the growth states of a tissue piece or a cultured cell of a plant in Steps (1) to (4) of Fig. 1.

## DETAILED DESCRIPTION OF THE INVENTION

As previously mentioned, the present invention involves the provision of not only a method for propagating a nursery plant but also a method for acclimatizing a cultured nursery plant.

In order to attain the above objects, the present in ventors forwarded their reseraches to find out that a nursery plant could be propagated preferably by starting the cultivation of a tissue piece or a cultured cell of a plant in a state of the tissue piece or the cultured cell being placed on a medium prepared by impregnating a water-insoluble and water-absorbent support with a liquid culture medium and carrying out the subsequent cultivation in all the processes of regeneration, rooting and acclimatization in the same culture tank and on the same medium.

3

In case of carrying out the cultivation consistently in the same culture tank and on the same medium as described above, a carbon source contained in a medium is consumed as the cultivation period proceeds to hinder the heterotrophical growth of a plant. Thus, it is considered that it becomes necessary to supply a carbon source to a medium at a suitable time during the cultivation period, from the conventional technical common sense described above. However, the present inventors found out that a plant after the regeneration of a shoot unexpectedly had a sufficient autotrophical growing ability already contrary to the conventional technical common sense and, in addition, the rooting ratio, the root taking ratio at the time of acclimatization, the growth of a plant after acclimatization, etc. were strikingly improved by carrying out the regeneration, the rooting and the acclimatization of a shoot under cultivation conditions which could positively stimulating the foregoing autotrophical growth.

Furthermore, the present inventors took note of the supplying of carbon dioxide into a culture tank as a requisite for giving the foregoing photo-autotrophical conditions in case of carrying out the cultivation under complete photo-autotrophical conditions in acclimatization process, and they confirmed through various experiments that this carbon dioxide gassing could replace the conventional means of lowering the humidity in the culture tank in the acclimatization process. Whereby, they accomplished the present method for acclimatizing a cultured nursery plant.

Hereinafter, the present methods will be described, referring to the attached drawings.

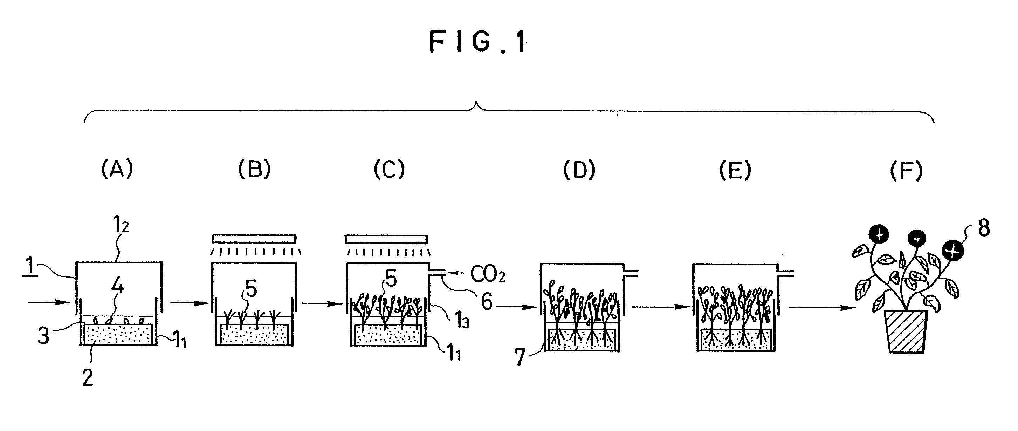

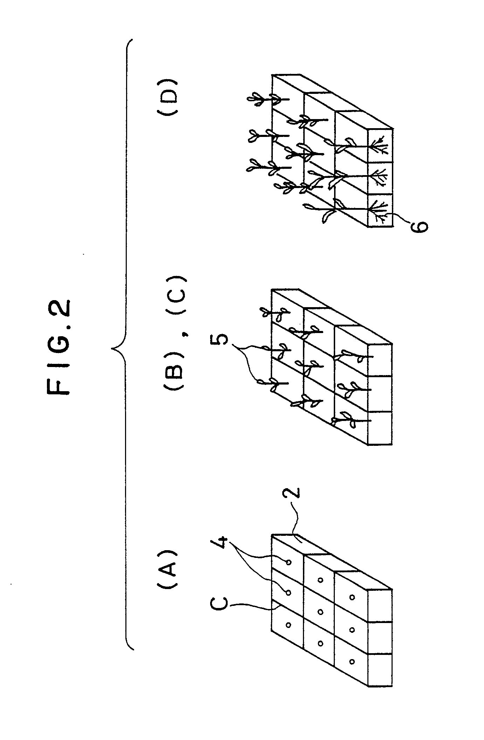

Fig. 1 is a schematic flow chart showing Steps (1) to (6) during the cultivation period according to the present method for propagating a nursery plant and Fig. 2 illustrates perspective views showing the growth states of a tissue piece or a cultured cell of a plant in Steps (1) to (4) of Fig. 1.

Fundamental features of the present method for propagating a nursery plant lie in starting the cultivation a tissue piece or a cultured cell of a plant 4 in a state of its being placed on a medium prepared by impregnating a water-insoluble and water-absorbent support 2 with a culture medium 3 [Step (1)] in a culture tank 1, carrying out the subsequent cultivation processes of regeneration [Steps (2) and (3)], rooting [Step (4)] and acclimatization [Step (5)] of a shoot 5 consistently in the same culture tank 1 and on the same medium 2 and, in the foregoing consistent cultivation, carrying out the cultivation of the tissue piece or the cultured cell of a plant 4 before the regeneration of the shoot 5 on a medium containing a carbon source heterotrophically and the subsequent cultivation in processes of rooting and acclimatization of the shoot 5 after the shoot regeneration [Step (2)] photo-autotrophically by making the shoot 5 photosynthesize under light exposure and carbon dioxide gassing conditions on the medium 2 where the carbon source is substantially consumed during the foregoing cultivation period before the shoot regeneration.

Furthermore, the present method for acclimatizing a cultured nursery plant is realized in Step (5) as a step of the foregoing consistent cultivation process.

The gassing of carbon dioxide into the culture tank 1 after the shoot regeneration can be carried out, for example, by changing a cap of the culture tank 1 from a sealing cap $1_2$ to a cap $1_3$ having a vent hole 6 for gassing carbon dioxide and then gassing carbon dioxide through the foregoing vent hole 6, as shown in Fig. 1. In addition, the making of cuts C in the water-absorbent support 2 at required intervals facilitates the division of individual nursery plants 8 after the completion of cultivation.

As a tissue piece or a cultured cell of a plant as a starting material for the present method for propagating a nursery plant, there is no particular restriction thereto] and thus explants which are used for the ordinary propagation of a plant, that is, a tissue piece, cullus, etc. of a flower organ, a stem, a leaf, a shoot apex, a bulb, a root, etc. can be enumerated.

A cultured nursery plant to be used in the present acclimatization method is a nursery plant obtained by the ordinary tissue culture of the above tissue piece or cultured cell of a plant.

As a water-insoluble and water-absorbent support, granular or fibrous carriers such as sterilized perlite, vermiculite, rockwool, polyester, soil, water-absorbent polymer granules, ceramic wool, etc. can be enumerated.

As a culture medium, the one prepared by adding amino acid or the like, on demand, to a basal medium such as Murashige-Skoog's medium, Nitch-Nitch's medium or the like is used.

As a culture medium to be impregnated into a water-insoluble and water-absorbent support at the start of cultivation, it is preferable to use the one containing a carbon source, for example, sucrose in such a quantity that the heterotrophical cultivation before the shoot regeneration can be accomplished to a required degree and that the carbon source in the medium is substantially consumed at the time of shoot regeneration, that is, about 0.5 to 1.5% carbon source. Whereby, the heterotrophical cultivation is substantially discontinued after the shoot regeneration and the shoot is autotrophically cultivated while making it photosynthesize under light exposure and carbon dioxide gassing conditions instead. In this case, it is preferred that the intensity of light exposure is in the range of 3,000 to 20,000 from the viewpoint of activating the photosynthesis. From the same viewpoint, it is preferred that carbon dioxide is gassed at a concentration of 300 to 1,200 ppm and a gas exchange ratio of 0.1 to 30 times/hour.

Incidentally, the gassing of carbon dioxide into a culture tank after the shoot regeneration is not necessarily carried out simultaneously with the start of light exposure at a required intensity [Step (2)] as shown in Fig. 1 and thus may be started at the time when leaves of a shoot 5 grow and the concentration of carbon dioxide residing in the culture tank from the beginning is lowered [Step (3)].

From the viewpoints of accelerating rooting and the like, it is preferred that the cultivation during the rooting period from Step (2) to Step (3) is carried out under a condition that the culture medium is free from auxin or con tains such an infinitesimal quantity of auxin that the rooting period of shoots may become concurrent. Specifically, it is preferred that the auxin content is in the range of $10^{-9}$ to $10^{-7}$.

With respect to the cultivation in the acclimatization period according to the conventional methods, a cultured nursery plant is transferred into an acclimatizing medium in a different culture tank and the cultivation thereof is carried out in parallel with gradual decreasing of the humidity. On the other hand, the cultivation in this period according to the present invention can be carried out in parallel with gradual decreasing of the moisture of an acclimatizing medium and the humidity of the culture tank by gassing carbon dioxide into the foregoing culture tank during the cultivation period to thereby discharge the inside moisture of the culture tank outside.

Although the present method for propagating a nursery plant and the present method for acclimatizing a cultured nursery plant can be applied to any plant, they are very suitable for the propagation of a nursery plant of a plant belonging to the genus *Rosa*.

As the foregoing plant belonging to the genus *Rosa*, plants belonging to large and medium flowered roses such as hybrid tea rose, grandiflora, floribunda, etc.; climbing roses such as climbing hybrid tea rose, rambler, etc.; and miniature roses such as Deep Red, Starina, Cinderella, etc. can be exemplified.

[Examples]

Hereinafter the present invention will be described, referring to examples. However, the present invention is nowise restricted to the examples.

Examples 1 to 3

A stem of Deep Red as a miniature rose was steriliezed, from which lateral buds were collected. The lateral buds were placed on perlite, rockwool and polyester fiber impregnated with a sterile Murashige-Skoog's liquid culture medium of pH 5.7 containing 1% sucrose and 0.1μM benzyl adenine, followed by cultivating at 25°C under light conditions of 16-hour light period (10,000 lux) and 8-hour dark period in a culture tank. On the 8th week from the start of cultivation, air containing 500 ppm carbon dioxide was supplied at a gas exchange ratio of 10 times/hour at the time when sucrose in the medium was consumed. On the 3rd week from the start of cultivation 0.01μM naphthaleneacetic acid was added to the medium. After continuing the cultivation for 4 weeks in total, nursery plants were taken out from the culture tank and then transplanted to soil. The results with respect to number of shoot, rooting ratio and root taking ratio per lateral bud were given in Table 1.

Comparative Example 1

Lateral buds of a Deep Red as a miniature rose were cultivated in the same manner as in Example 1, except that a medium solidified by agar was used, the light intensity at the light period was set at 1,000 lux and carbon dioxide was not gassed. The results were given in Table 1.

Comparative Example 2

Lateral buds of a Deep Red as a miniature rose were cultivated in the same manner as in Example 1, except that a liquid culture medium was used, oxygen was gassed, the light intensity at the light period was set at 1,500 lux and carbon dioxide was not gassed. The results were given in Table 1.

Table 1

| | Medium | Light Intensity | Number of Shoot | Rooting Ratio | Root Taking Ratio |
|---|---|---|---|---|---|
| Example 1 | Perlite + Liquid medium | 10,000 lux | 5.6 | 96 | 92 |
| Example 2 | Rockwool + Liquid medium | 10,000 lux | 5.4 | 98 | 94 |
| Example 3 | Polyester + Liquid medium | 10,000 lux | 5.2 | 94 | 90 |
| Comparative Example 1 | Agar medium | 1,000 lux | 3.4 | 76 | 60 |
| Comparative Example 2 | Liquid medium + Oxygen gassing | 1,500 lux | 3.6 | 56 | 45 |

**Claims**

1. A method for propagating a nursery plant, characterized in that the cultivation of a tissue piece or a cultured cell of a plant in a culture tank is started by placing the tissue piece or cultured cell on a medium prepared by impregnating a water-insoluble and water-absorbent support with a culture medium and in that all the subsequent cultivation processes of regeneration, rooting and acclimatization of a shoot are carried out in the same culture tank and on the same medium.

2. A method according to claim 1, which comprises heterotrophically cultivating said tissue piece or cultured cell on a medium containing a carbon source before the regeneration of a shoot and photo-autotrophically cultivating the regenerated shoot in the processes of rooting and acclimatization by making the shoot photosynthesize under a required light exposure intensity and a required carbon dioxide gassing condition on the medium from which the carbon source has been substantially consumed during the heterotrophic cultivation period before the regeneration of the shoot.

3. A method according to claim 2, wherein the intensity of light exposure is 3,000 to 20,000 lux.

4. A method according to claim 2 or 3, wherein the carbon dioxide is gassed at a concentration of 300 to 1,200 ppm and at a gas exchange ratio of 0.1 to 30 times/hour.

5. A method according to any one of claims 2 to 4, wherein the cultivation in the rooting period is carried out on an auxin-free medium or a culture medium containing such an infinitesimal quantity of auxin that the rooting period of shoots may become concurrent.

6. A method according to claim 5, wherein the auxin content is $10^{-9}$ to $10^{-7}$ M.

7. A method according to any of claims 2 to 6, wherein the cultivation in the acclimatization period is carried out in parallel with a gradual decrease in the moisture content of acclimatizing medium and the humidity in the culture tank by gassing carbon dioxide into the culture tank during the foregoing cultivation period to thereby discharge the moisture inside the culture tank to the outside.

8. A method according to any one of claims 1 to 7, wherein the water-insoluble and water-absorbent support is composed of a granular or fibrous carrier.

9. A method according to any one of claims 1 to 8, wherein the plant belongs to the genus Rosa.

10. A method for acclimatizing a cultured nursery plant, characterized by cultivating a cultured nursery plant on a carbon source-free acclimatization medium in a culture tank while making the cultured nursery plant photosynthesize under a required light exposure intensity and a required carbon dioxide gassing condition and, at the same time, gradually decreasing the moisture of the acclimatization medium and the humidity of the culture tank by gassing carbon dioxide into the culture tank during the foregoing cultivation period to thereby discharge the moisture inside the culture tank to the outside.

11. A method according to claim 10, wherein the light exposure intensity is 3,000 to 20,000 lux.

12. A method according to claim 10 or 11, wherein the carbon dioxide is gassed at a concentration of 300 to 1,200 ppm and at a gas exchange ratio of 0.1 to 30 times/hour.

13. A method according to any one of Claims 10 to 13, wherein the acclimatization medium is prepared by impregnating a water-insoluble and water-absorbent support with a required quantity of culture medium.

# FIG.1

EP 0 344 992 A1

(A)       (B)       (C)       (D)       (E)       (F)

# FIG.2

(A)　　　　　(B) , (C)　　　　(D)

EP 0 344 992 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4 ) |
|---|---|---|---|
| A | EP-A-219133 (DNA PLANTTECHNOLOGY CORPORATION) <br> * column 2, line 26 - column 8, line 22 * | 1-3, 7, 8, 10, 11, 13 | A01G7/00 |
| A | EP-A-245898 (SHELL INTERNATIONALE RESEARCH MAATSCHAPPIJ BV) <br> * page 3, line 1 - page 4, line 1 * | 1-3 | |
| A | US-A-3941662 (MUNDER ET AL) <br> * column 1, line 44 - column 2, line 9; claims 1, 7 * | 1-4, 10, 12 | |
| A | WO-A-8700394 (NIPPON STEEL CHEMICAL CO. LTD.) <br> * page 3, line 1 - page 9, line 21 * | 1, 7, 8, 13 | |
| A | US-A-4586288 (WALTON) <br> * column 3, line 35 - column 7, line 23; figures 1-7 * | 1, 2, 8 | |
| A | EP-A-31985 (REGENCY INVESTMENTS LTD.) <br> * page 6, line 10 - page 12, line 2; figures 1-6 * | 1 | |

**TECHNICAL FIELDS SEARCHED (Int. Cl.4 )**

A01H
A01G

The present search report has been drawn up for all claims

| Place of search | Date of completion of the search | Examiner |
|---|---|---|
| THE HAGUE | 15 SEPTEMBER 1989 | DISSEN H.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)